# EUROPÄISCHE PATENTSCHRIFT

(11) **EP 1 289 422 B1**
(45) Veröffentlichungstag und Bekanntmachung des Hinweises auf die Patenterteilung: **12.01.2011**
(21) Anmeldenummer: 01953956.8
(22) Anmeldetag: 25.05.2001
(51) Int. Cl.: A61B 5/15

(54) **SYSTEM ZUR ENTNAHME VON KÖRPERFLÜSSIGKEIT, INSBESONDERE BLUT**
SYSTEM FOR REMOVING BODY FLUID
SYSTEME DE PRELEVEMENT DE LIQUIDES CORPORELS

(30) Priorität: 26.05.2000 DE 10026172
(43) Veröffentlichungstag der Anmeldung: 12.03.2003
(73) Patentinhaber: Roche Diagnostics GmbH, 68305 Mannheim (DE); F.Hoffmann-La Roche AG, 4070 Basel (CH)
(72) Erfinder: SCHMELZEISEN-REDEKER, Guenther, 64653 Lorsch (DE); WEISS, Thomas, 68307 Mannheim (DE); DECK, Frank, 67150 Niederkirchen (DE); EBERT, Klaus-Peter, 64407 Fraenkisch-Crumbach (DE)
(86) Internationale Anmeldenummer: PCT/EP2001/005976
(87) Internationale Veröffentlichungsnummer: WO 2001/089383

(56) Entgegenhaltungen:
- US-A- 2 646 799
- US-A- 3 626 929
- US-A- 5 624 458
- US-A- 5 709 699

## Beschreibung

Die vorliegende Erfindung betrifft ein System zur Entnahme von Körperflüssigkeit aus einem Körperteil, insbesondere der Fingerbeere. Eine Entnahme von Körperflüssigkeiten wird vor allem mit dem Ziel einer nachfolgenden Analyse durchgeführt, um eine Diagnose von Krankheiten oder die Überwachung des Stoffwechselzustandes eines Patienten zu ermöglichen. Insbesondere von Diabetikern wird eine solche Entnahme durchgeführt, um die Blutzuckerkonzentration zu bestimmen. Ziel einer solchen, in der Regel täglich mehrfach durchgeführten Blutzuckerkontrolle ist es, sowohl hypoglykämische Zustände als auch hyperglykämische Zustände zu vermeiden. Bei einer Hypoglykämie kann der Patient in ein Koma verfallen und sogar sterben, da das Gehirn nicht mehr ausreichend mit Glucose versorgt wird. Hyperglykämische Zustände hingegen können zu Spätschäden wie Erblindungen, Gangränen und dergleichen führen.

Eine häufige Kontrolle des Blutzuckerspiegels ist demnach eine unumstrittene Notwendigkeit. Es liegt daher auf der Hand, daß ein dringender Bedarf an Entnahmesystemen besteht, die für den Benutzer einfach zu handhaben und vor allem schmerzarm sind.

Im Stand der Technik sind bereits seit einiger Zeit Blutentnahmesysteme bekannt, mit denen der Patient oder Krankenhauspersonal eine Entnahme auf einfache Weise durchführen kann. Ein hierfür geeignetes Gerät ist beispielsweise das im Handel befindliche Softclix, dessen Funktionsweise in der US 5,318,584 beschrieben ist. Dieses Gerät sieht eine Einstellmöglichkeit für die Einstechtiefe einer Lanzette in das Gewebe vor. Der Patient kann so die minimale Einstechtiefe wählen, mit der er eine für eine nachfolgende Analyse gerade noch ausreichende Blutmenge erhält und dadurch den Einstichschmerz sehr niedrig halten. Nachdem der Patient eine Hautöffnung durch Einstich erzeugt hat, muß er, insbesondere bei geringen Einstechtiefen, den Finger massieren bzw. drücken, um ausreichend Blut aus der Stichwunde herauszubefördern. Diese, von Diabetikern häufig als "melken" bezeichnete Operation, kann bisher lediglich bei einem tiefen Einstich vermieden werden, der für den Patienten entsprechend unangenehm ist und zu einer starken Vernarbung an den empfindlichen Fingerkuppen führen kann. Im Stand der Technik bekannte Vorrichtungen versuchen, den Blutaustritt durch Anlegen eines Vakuums zu fördern, was sich aber als wenig effizient erwiesen hat.

Im Stand der Technik sind weiterhin Vorrichtungen bekannt, bei denen ein sogenannter Stimulator mit einem Ring die Hautoberfläche niederdrückt, der eine Einstichstelle umgibt. Eine solche Vorrichtung zur Gewinnung von interstitieller Flüssigkeit ist beispielsweise in der US 5,582,184 beschrieben. Der verwendete Ring zum Niederdrucken der Hautoberfläche ist aus einem starren Material gefertigt. Mit dem Gerat können lediglich geringe Flüssigkeitsmengen gewonnen werden, die für handelsübliche Analysensysteme nicht ausreichen.

Das Dokument US 5,709,699 beschreibt eine Stechvorrichtung, bei der die Nadel fest im System positioniert ist. Als Fingerauflagefläche um die Nadel herum dient eine komprimierbare Kappe, die vom Benutzer zusammengedrückt werden kann. Hierbei wird der Finger von dem Benutzer gegen das Nadelelement gedrückt, so dass ein Einstich erfolgt.

Aus der US 5,857,983 ist weiterhin eine Vorrichtung bekannt, bei der mit einer Kanüle in die Hautoberfläche eingestochen wird und die die Einstichstelle umgebende Hautoberfläche mit einem sogenannten Simulator wiederholt niedergedrückt wird, um Körperflüssigkeit in die Kanüle zu drücken. Bei dieser Vorrichtung wird analog dem zuvor genannten Dokument ein starrer Ring verwendet, um die Hautoberfläche niederzudrücken. Auch die mit dieser Vorrichtung erzielbaren Mengen an Körperflüssigkeit sind gering und somit für herkömmliche Analysensysteme nicht ausreichend.

In dem US Patent 5,951,493 sind ebenfalls Blutentnahmevornchtungen beschrieben, die mit einem Stimulator wie bei dem vorstehend genannten US Patent arbeiten In den Figuren 15 his 17 ist ferner eine Vorrichtung beschrieben, bei der der Bereich der Vorrichtung, der zum Andruck an eine Körperoberfläche dient, mit Hebeln (104) versehen ist, die eine Hautpartie lateral zusammendrücken , während die Vorrichtung auf die Körperoberfläche aufgedrückt wird. Die in diesem Patent beschriebenen Vorrichtungen sind insbesondere zur Entnahme von Körperflüssigkeiten an anderen Orten als der Fingerbeere vorgesehen. Weiterhin ist dem Dokument zu entnehmen, daß die Beförderung von Körperflüssigkeit auf die Hautoberfläche durch wiederholtes Andrücken der Vorrichtung erzielt wird.

Aus dem Dokument US 3,626,929 ist eine Vorrichtung bekannt, bei der der Finger vor einer Blutentnahme zwischen einen Hebel und einer Fingerauflage eingeklemmt wird. Die Fingerauflage wird durch einen Motor bewegt, um eine Massierung proximal der Einstichstelle zu bewirken. Zur Entnahme wird der Finger vom Benutzer auf eine flexible Kappe gedrückt, in der sich Nadeln und ein Fluidkanal befinden. Ungünstig ist bei dieser Vorrichtung, daß die Nadeln zur Entnahme im Körper verbleiben und. daß in diesem Zustand eine Bewegung der Fingerauflage erfolgt. Hierdurch wird nämlich eine Bewegung der Nadeln im Finger hervorgerufen. was in der Regel zu erheblichem Schmerz führt. Außerdem ist es höchst unwahrscheinlich, daß Blut austritt, während sich eine Nadel im Finger befindet, da der Kanal durch die Nadel verschlossen ist. In den Figuren 11 und 12 ist ein Sammelbehälter dargestellt, dem einen flexiblen Andruckbereich aufweist. Aufgrund der Form des Andruckbereiches, der sich zum Finger konisch erweitert, findet jedoch keine Umsetzung einer primären Andruckbewegung in eine laterale Bewegung statt, die den Entnahmebereich zusammendrückt.

Die Aufgabe der vorliegenden Erfindung war es, ein System zur Entnahme von Körperflüssigkeiten zur Verfügung zu stellen, das bei geringen Einstichtiefen eine ausreichende Menge an Körperflüssigkeit, insbesondere Blut liefert Weiterhin war es Aufgabe der Erfindung, ein System anzubieten. das einfach angewandt werden kann und das mit geringem apparativem Aufwand auskommt. Einfachheit in diesem Sinne bedeutet insbesondere, daß die Zahl der Handhabungsschritte möglichst klein ist.

Als weitere Aufgabe der Erfindung ist es zu nennen, insbesondere die Blutentnahme an der Fingerbeere zu vereinfachen und hierbei den unterschiedlichen Fingergrößen sowie den unterschiedlichen Punktionsstellen an der Fingerspitze Rechnung zu tragen.

Die vorliegende Erfindung betrifft demnach ein System zur Entnahme von Körperflüssigkeit aus einem Körperteil, insbesondere der Fingerbeere, mit den folgenden Elementen:
- einer Kompressionseinheit, an die das Körperteil in einer Primärrichtung angedrückt wird und die den Andruck teilweise in eine Bewegung in eine Sekundärrichtung mit Anteil senkrecht zur Primärrichtung umwandelt, so daß eine Erhöhung des Innendruckes in einem Bereich des Körperteiles erzeugt wird,
- einer Perforationsvorrichtung, insbesondere einer Lanzette oder Kanüle, zur Erzeugung einer Körperöffnung in dem Bereich erhöhten Innendruckes,
wobei die Kompressionseinheit einen Andruckbereich aus einem deformierbaren Material besitzt und in das System ein Analysesystem zur Bestimmung der Konzentration eines Analyten, inbesondere Glucose, integriert ist

Durch Verwendung der erfindungsgemäßen Kompressionseinheit kann auf einfache und für den Benutzer angenehme Weise die oben genannte Melkbewegung zum Herausdrücken von Blut aus der Einstichstelle nachgeahmt werden. Die Kompressionseinheit liefert nicht nur größere Mengen an Körperflüssigkeit als dies mit den Andruckvorrichtungen des Standes der Technik der Fall ist, sondern der Andruck- und Entnahmevorgang ist für den Patienten auch deutlich angenehmer. Dies liegt zum einen daran, daß sich die Kompressionseinheit dem Körperteil, insbesondere einem Finger anschmiegt. Darüber hinaus kommt aber auch zum tragen, daß es aufgrund der Kompressionseinheit möglich ist, bereits mit sehr geringen Einstechtiefen ausreichende Mengen an Körperflüssigkeit zu gewinnen.

Ein weiterer sehr wichtiger Vorteil der vorliegenden Erfindung besteht darin, daß es durch Verwenden einer Kompressionseinheit mit einem Andruckbereich aus deformierbarem Material möglich ist, mit der Kompressionseinheit zuverlässig und angenehm Körperflüssigkeit aus verschiedenartig gestalteten Körperteilen zu entnehmen. Insbesondere ist so eine Entnahme aus unterschiedlich großen Fingern leicht und zuverlässig möglich. Außerdem werden durch das deformierbar Material Formunterschiede des angedrückten Körperteiles (Fingerkuppe versus Fingerseite) aufgefangen.

Mit einem erfindungsgemäßen System kann besonders vorteilhaft Kapillarblut aus der Fingerbeere gewonnen werden. Darüber hinaus ist auch eine Entnahme von Blut oder interstitieller Flüssigkeit aus anderen Körperteilen, wie zum Beispiel dem Arm, möglich.

Ein wesentliches Element des Systems ist die Kompressionseinheit, die dazu führt, daß das Zusammendrücken des Körperteiles nicht nur in Richtung des primären Andruckes erfolgt, sondern daß der Andruck zumindest zum Teil auch umgelenkt wird, so daß ein Zusammendrücken mit Kraftkomponenten quer zur primären Andruckrichtung auftritt. Der Bereich des Körperteiles, aus dem eine Entnahme erfolgen soll, wird hierdurch lateral zusammengedrückt. Die Wirkungsweise dieser vorteilhaften Kompression wird später anhand der Ausführungsbeispiele näher erläutert. Durch die Kompressionseinheit wird eine Erhöhung des Innendruckes in einem Bereich des Körperteiles erzeugt. Dieser Bereich erhöhten Innendruckes ist dem Bereich, auf den der Andruck wirkt, benachbart oder er ist von dem Andruckbereich umgeben. Aus dem Bereich erhöhten Innendruckes kann nunmehr mit einer Perforationsvorrichtung eine Perforation erfolgen und Körperflüssigkeit entnommen werden.

Die Kompressionseinheit beinhaltet einen Andruckbereich aus einem deformierbaren Material. Ein solches Material macht einerseits den Entnahmevorgang für den Benutzer angenehmer andererseits kann so auch leichter eine Anpassung an unterschiedlich geformte oder unterschiedlich große Körperteile erfolgen. Als Material des Andruckbereiches können beispielsweise deformierbare Kunststoffe wie Elastomere, Gummi und dergleichen dienen. Vorzugsweise ist der Andruckbereich ringförmig ausgebildet. Dies stellt einen technisch bedeutsamen Unterschied zu der in den Figuren 15 bis 16 der US 5,951,493 beschriebenen Vorrichtung dar. Bei der Vorrichtung des Standes der Technik wird eine Anordnung von separaten Hebelarmen zum lateralen Zusammendrücken der Körperoberfläche verwendet. Dies hat eine Reihe von Nachteilen. Durch den Abstand der Hebelarme voneinander erfolgt das laterale Zusammendrücken der Hautoberfläche nur unvollständig, da Bereiche zwischen den Hebelarmen unberührt bleiben. Weiterhin kann schlimmstenfalls ein Verklemmen von Haut zwischen den Hebelarmen erfolgen, während sich diese aufeinander zubewegen. Schließlich ist noch anzumerken, daß die Vorrichtung des Standes der Technik weder dazu ausgelegt noch dazu geeignet ist, sich an unterschiedlich große oder unterschiedlich geformte Körperoberflächen anzuschmiegen. Die Vorrichtung ist primär dazu gedacht, eine Entnahme von Körperflüssigkeit aus einem im Vergleich zur Fingerbeere wenig gekrümmten Bereich am Arm zu entnehmen. Die genannten Probleme können durch Verwendung eines Andruckbereiches aus einem deformierbaren Material, das vorzugsweise die Form eines Ringes aufweist, vermieden werden.

Das System zur Entnahme von Körperflüssigkeit weist weiterhin eine Perforationsvorrichtung zur Erzeugung einer Körperöffnung auf Eine solche Perforationsvorrichtung kann eine Lanzette oder Kanüle sein.

Im Falle einer Lanzette wird diese nach dem Einstich vorzugsweise ganz aus dem Gewebe entfernt, so daß die Einstichstelle aus der die Körperflüssigkeit austritt, zugänglich wird. Im Falle einer Kanüle kann diese in der maximalen Einstechtiefe verharren, um aus dieser Tiefe Körperflüssigkeit zu fördern, oder aber bis auf die Hautoberfläche zurückgezogen werden, um von hier Flüssigkeit aufzunehmen. Es ist auch möglich, die Kanüle nur teilweise zurückzuziehen, so daß einerseits der Stichkanal zum Teil freigemacht wird, die Kanüle jedoch noch in der Haut verbleibt. Hierdurch kann einerseits durch den freiliegenden Stichkanal besser Flüssigkeit austreten, andererseits ist keine Positionierung der Kanüle auf der Körperoberfläche zur Aufnahme notwendig.

Die Perforationsvorrichtung kann vorzugsweise durch eine Öffnung in der Kompressionseinheit bzw. im Andruckbereich an die Körperoberfläche herangeführt werden. Zur Erzielung unterschiedlich tiefer Stiche, um den verschiedenen Hauttypen bzw. auch den erforderlichen Blutmengen Rechnung zu tragen, ist es von Vorteil, wenn die Einstechtiefe variabel ist. Zur Erzielung einer definierten (und ggf. vorher eingestellten) Einstechtiefe hat es sich als besonders günstig erwiesen, die Perforationsvorrichtung gegenüber der Kompressionseinheit verschiebbar anzuordnen. Diese Verschiebbarkeit umfaßt zwei Ausgestaltungen, die auch miteinander kombiniert werden können. Bei der ersten Ausgestaltung ist die Perforationsvorrichtung relativ zur Kompressionseinheit federgelagert, so daß eine Frontfläche oder ein Anschlag der Perforationsvorrichtung federnd an der Körperoberfläche anliegt, wenn das Körperteil an die Kompressionseinheit angepresst wird. Die Andruckkraft der Perforationsvorrichtung an die Körperoberfläche liegt vorteilhaft im Bereich von 1 bis 5 N und beträgt vorzugsweise ca. 2 N. Werden größere Andruckkräfte verwendet, so besteht die Gefahr, daß Körperflüssigkeit, die sich im Bereich erhöhten Innendruckes befindet, aus diesem Bereich herausgedrückt wird.

Der zweite Aspekt der relativen Verschiebbarkeit von Perforationsvorrichtung und Kompressionseinheit betrifft ein Hinbewegen der Perforationsvorrichtung in eine Perforationsposition und ein Wegbewegen aus dieser Position, um Platz für eine Aufnahme von Körperflüssigkeit durch eine Aufnahmevorrichtung zu schaffen. Dieser Aspekt ist besonders wichtig für eine integrierte Anordnung, mit der sowohl eine Blutentnahme als auch eine Analyse durchgeführt wird.

Bei der Erfindung ist in das System zur Entnahme von Körperflüssigkeit ein Analysesystem integriert. Derartige Analysesysteme sind im Stand der Technik hinlänglich bekannt. Im Handel befinden sich beispielsweise Analysensysteme mit der Bezeichnung Accucheck Plus^{®} und Accucheck Advantage^{®}. Im Regelfall arbeiten Analysensysteme, die für den Consumerbereich konzipiert sind, mit disposiblen Testelementen, die nach Kontaktieren mit einer Probenflüssigkeit ein Signal in Abhängigkeit von der Analytkonzentration abgeben. Im Bereich der Blutzuckermessung werden sowohl optische Testelemente verwendet, bei denen die Reaktion von Glucose mit einer Testchemie zu einer Farbveränderung führt als auch elektrochemische Testelemente, bei denen eine enzymatische Umsetzung von Glucose eine amperometrische oder potentiometrische Analyse ermöglicht. Die Testelemente können vorteilhaft so ausgestaltet sein, daß sie Körperflüssigkeit aktiv (z. B. über einen Kapillarspalt) aufnehmen.

Das erfindungsgemäße System vereinfacht die Integration einer Entnahmeeinheit mit einem Analysesystem oder ermöglicht eine Integration überhaupt erst. Wie bereits ausgeführt wurde, ist im Stand der Technik üblich, die Körperflüssigkeit nach Erzeugung einer Hautöffnung manuell herauszupressen, was damit verbunden ist, daß der Patient das Körperteil von der Entnahmevorrichtung entfernt. Mit einem System gemäß der vorliegenden Erfindung ist es hingegen möglich, daß der Patient das Körperteil an die deformierbare Kompressionseinheit andrückt und dort in diesem angedrückten Zustand sowohl zum Erzeugen einer Hautöffnung als auch zur Entnahme beläßt. Es ist so ein stärkerer Automatisierungsgrad möglich, bei dem der Patient lediglich das Andrücken an die Kompressionseinheit übernimmt und alle nachfolgenden Schritte bis hin zur Ausgabe des Analyseergebnisses automatisch ablaufen können.

Eine Integration von Perforationsvorrichtung und Analysesystem betrifft somit vorteilhaft nicht nur eine räumliche Integration sondern auch eine verfahrensmäßige Integration, die es ermöglicht, Handhabungsschritte seitens des Benutzers zu vermeiden. Dementsprechend besitzt ein solches System vorteilhaft auch eine Steuereinheit, die eine Aktivierung der Entnahmevorrichtung, die Entnahme von Körperflüssigkeit bzw. den Transport von Körperflüssigkeit zum Analysesystem und die Analyse koordiniert steuert.

Ausgestaltungsmöglichkeiten von Systemen gemäß der vorliegenden Erfindung werden anhand von Figuren näher erläutert:
- Figur 1:: Kompressionseinheit in perspektivischer und Querschnittsansicht
- Figur 2:: Kompression eines Bereiches der Fingerkuppe in perspektivischer und Quer- schnittsansicht.
- Figur 3:: Weg-Kraft-Diagramm für den Andruck eines Fingers an eine Kompressionseinheit gemäß Figur 1
- Figur 4:: Querschnittsdarstellung einer weiteren Ausgestaltung einer Kompressionseinheit
- Figur 5:: Integriertes System zur Analyse von Körperflüssigkeiten mit einer Kompressions- einheit gemäß Figur 1
- Figur 6:: Integriertes System zur Analyse von Körperflüssigkeiten beinhaltend eine Kom- pressionseinheit, eine Perforationseinheit sowie eine Analyseeinheit

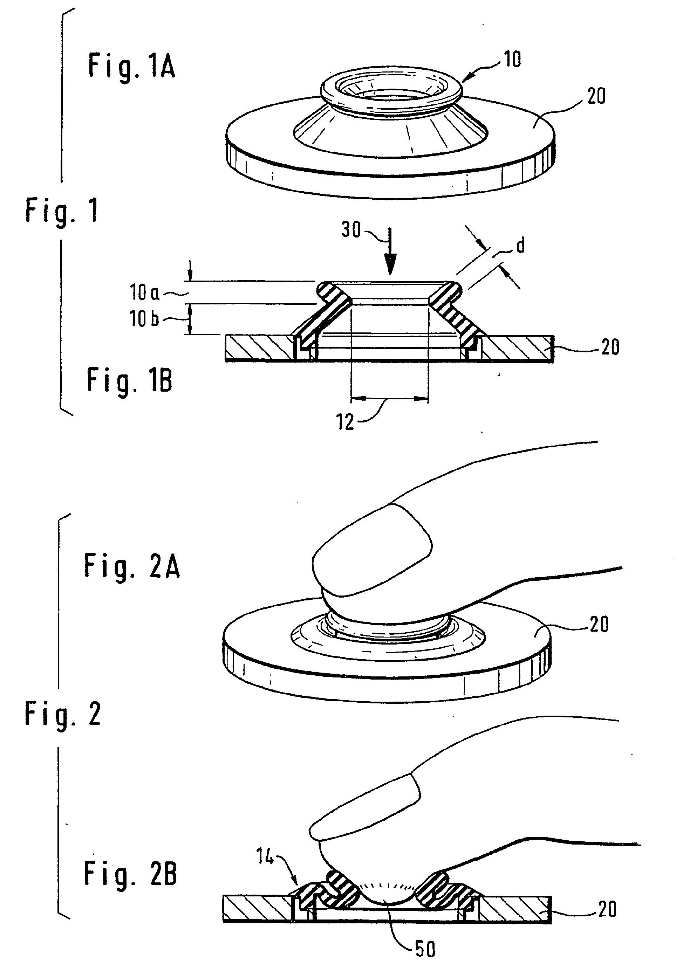

Figur 1 zeigt eine erste Ausführungsform einer deformierbaren Kompressionseinheit.
Figur 1A zeigt eine perspektivische Darstellung der Kompressionseinheit (10), die auf einer Platte (20) angebracht ist. Aus der Querschnittsdarstellung Figur 1B ist zu entnehmen, daß die Kompressionseinheit zwei konisch zulaufende Bereiche (10a, 10b) besitzt, die als Andruckbereich fungieren. Der obere Andruckbereich (10a) verengt sich in Richtung der Platte (20) und der sich direkt anschließende, untere Bereich (10b) erweitert sich in Richtung der Platte. Die Kompressionseinheit ist aus einem deformierbaren Kunststoff, im dargestellten Beispiel Polyurethan, aufgebaut. Aber auch Silikone und Gummi sind möglich. Entscheidend ist es, daß die Kompressionseinheit sowohl ausreichend leicht deformierbar als auch ausreichend fest ist, um den notwendigen Gegendruck und einen ausreichenden Druck in Querrichtung zu erzeugen. Geeignet sind insbesondere Materialien mit einer Härte von weniger als 90 Shore bevorzugt weniger als 50 Shore, besonders bevorzugt im Bereich von 20 bis 40 Shore.

Durch die Struktur, die im Querschnitt die Form zweier aufeinander zugerichteter Pfeile besitzt und durch die Deformierbarkeit wird eine primäre Bewegung (30) eines Körperteiles senkrecht zur Platte (20) zumindest teilweise in eine quer dazu verlaufende Bewegung umgewandelt. Durch die so entstehende Sekundärbewegung erfolgt ein Zusammendrücken des Körperteiles, das die vom Benutzer sonst manuell durchgeführte Melkbewegung nachahmt.

Mit der in Figur 1 dargestellten Kompressionseinheit, die eine Shore-Härte von 30 besitzt, war es bei mehreren Patienten bereits bei einer Einstechtiefe von 0,7 mm möglich, eine Blutmenge von 1,5 - 3 µl aus der Fingerbeere zu gewinnen. Die gewonnene Blutmenge wird im wesentlichen durch das mit der Kompressionseinheit zusammengedrückte Gewebevolumen bestimmt. Aufgrund dieser Beschränkung ist die Blutmenge begrenzt, so daß Hygieneprobleme durch zu großen Blutaustritt vermieden werden können.

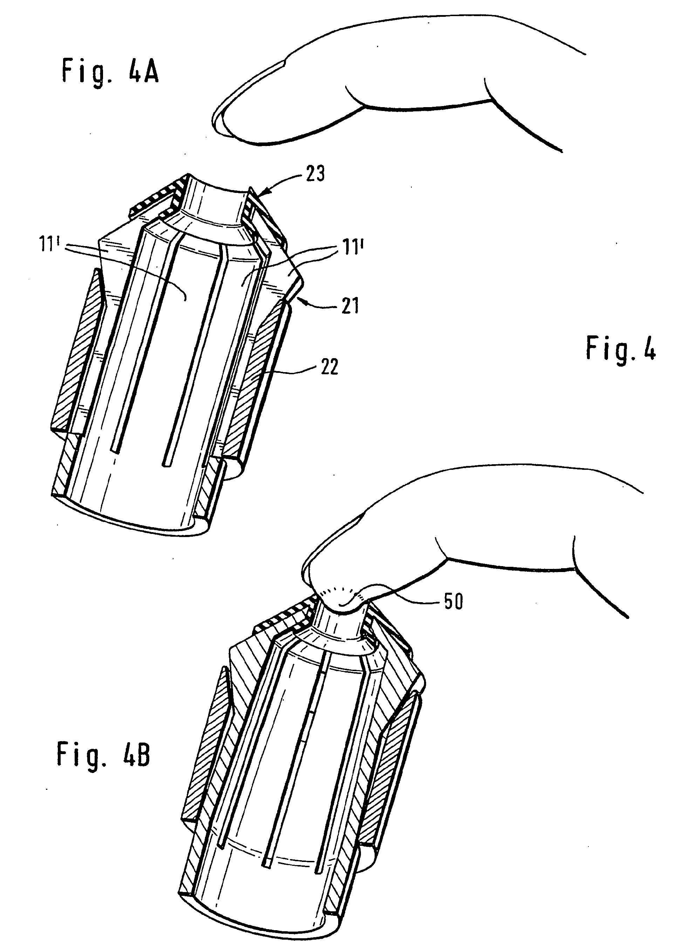

Von der vorliegenden Erfindung sollen Anordnungen umfaßt sein, bei denen eine Primärbewegung des Körperteiles relativ zur Kompressionseinheit zumindest teilweise in eine Bewegung umgesetzt wird, die quer dazu verläuft. So soll zum Beispiel auch ein System umfaßt sein, bei dem der obere Bereich (10a) der Kompressionseinheit fehlt und die Umsetzung der Primär- in eine Sekundärbewegung lediglich durch den unteren Teil (10b) erfolgt. Der Begriff deformierbar soll dabei nicht nur solche Ausführungsformen umfassen, deren Material in sich deformierbar ist, wie dies bei der in Figur 1 dargestellten Ausführungsform der Fall ist, sondern es sollen auch solche Ausführungsformen eingeschlossen sein, deren geometrische Anordnung durch die Primärbewegung deformiert bzw. umgestaltet wird. Ein Beispiel für eine derartige Ausführungsform wird im folgenden in Zusammenhang mit Figur 4 beschrieben.

Figur 2 zeigt die Betätigung und Wirkung der Kompressionseinheit aus Figur 1. Wie aus Figur 2A zu erkennen ist, wird vom Benutzer ein Körperteil, vorzugsweise ein Fingerende auf die Kompressionseinheit gedrückt, so daß die Kompressionseinheit zusammengedrückt wird und sich die lichte Weite (12) der Kompressionseinheit verringert. Hierdurch wird ein Stück des Fingerendes eingequetscht und der Innendruck in diesem Bereich (50) erhöht. Die lichte Weite (12) sollte idealerweise im Bereich von 8 bis 11 mm liegen, um sowohl für große Erwachsenenfinger als auch Kinderfinger geeignet zu sein.

Aus Figur 2b ist weiterhin zu erkennen, daß bei dieser Ausführungsform der Andruckbereich (10a, 10b) nicht nur eine Sekundärbewegung parallel zur Platte (20) ausführt sondern auch nach unten gewölbt wird. Diese Deformationsbewegung der Kompressionseinheit verstärkt den Effekt des Zusammenpressens innerhalb des eingeklemmten Körperbereiches und trägt so zur gewünschten Erhöhung des Innendruckes bei. Ein besonderer Vorteil der in Figur 1 dargestellten Kompressionseinheit mit einem Andruckbereich aus deformierbarem Material ist es, daß sie sich der Kontur des Körperteiles anpaßt. Es ist so beispielsweise möglich, auch Blut aus einer Seite der Fingerkuppe zu entnehmen, was mit starren Andruckvorrichtungen quasi unmöglich wäre.

Durch eine geeignete Wahl der Wandstärken bzw. der Härte des Materials kann das beim Andrücken der Kompressionseinheit auftretende partielle Umklappen der Kompressionseinheit vom Benutzer deutlich gespürt werden. Auf diese Weise kann der Benutzer spüren, ob der Andruck für die Blutgewinnung ausreichend ist. Das prinzipielle Funktionsprinzip der erfindungsgemäßen Kompressionseinheit bleibt jedoch auch ohne ein solches spürbares Umklappen der Anordnung gültig.

Aufgrund der Figur 2 ist zu erkennen, daß in dem Bereich 14, in dem die Kompressionseinheit (10) mit der Platte (20) verbunden ist, im angedrückten Zustand hohe Zugspannungen auftreten. Es hat sich erwiesen, daß diese Zugspannungen sehr günstig für die Funktionsweise der Anordnungen sind. Somit ist es vorteilhaft, für einen guten Kraftschluß zwischen der Kompressionseinheit und der Platte zu sorgen und insbesondere die beiden Komponenten im Bereich (14) haltbar miteinander zu verbinden.

Ein weiteres Detail, das aus Figur 2 zu erkennen ist, ist die Bedeutung der Dicke (d) des Andruckbereiches. Im angedrückten Zustand liegen oberer und unterer Andruckbereich aneinander und führen so zu einer noch stärkeren Kompression des Körperteiles in der Kompressionseinheit.

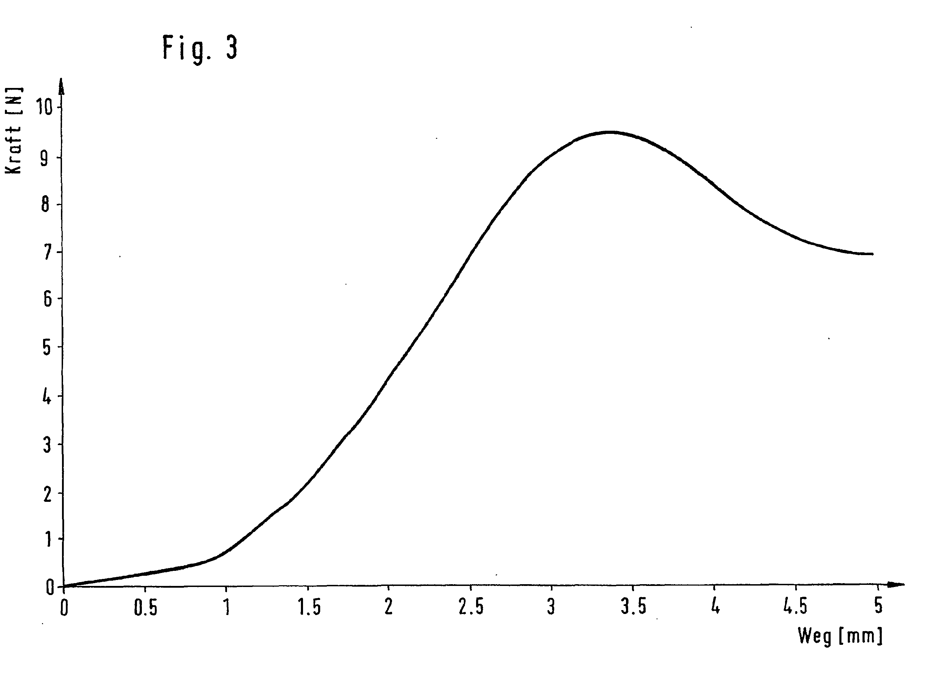

Der vorstehend bereits genannte Umklappeffekt der Kompressionseinheit ist aus Figur 3 zu erkennen. Auf der Abszisse ist der Weg einer Fingerkuppe relativ zur Kompressionseinheit in mm dargestellt. Auf der Ordinate findet sich die Abstoßungskraft zwischen Finger und Kompressionseinheit. Aus Figur 3 ist zu erkennen, daß beim beginnenden Andrücken des Fingers die Abstoßungskraft anwächst, ein Maximum durchläuft und bei weiterer Annäherung wieder abfällt. Die Position 5 mm entspricht dabei dem in Figur 2 dargestellten Zustand. Das Überwinden des Kraftmaximums wird vom Benutzer als ein Umklappen empfunden, das ihm zugleich signalisiert, daß er seinen Finger ausreichend fest und weit an die Andruckeinheit herangeführt hat. Das Durchlaufen eines Kraftmaximums ist ebenfalls vorteilhaft, weil der Benutzer auf diese Weise veranlaßt wird, seinen Finger für den Zeitraum der Perforation und ggf. Blutentnahme angedrückt an die Kompressionseinheit zu belassen.

Figur 4 zeigt eine den Figuren 1 und 2 ähnliche Ausführungsform, bei der die Kompressionseinheit jedoch aus an sich starren Elementen aufgebaut ist, die durch den Andruck des Körperteiles abgebogen werden, so daß die geometrische Struktur der Kompressionseinheit insgesamt deformiert wird. Die Kompressionseinheit der Figur 4 weist eine Vielzahl von Lamellen (11') auf, die etwas voneinander beabstandet sind. Die Lamellen besitzen an ihrer Außenseite eine Schräge (21) durch die sie aufeinander zubewegt werden, wenn die Einheit aus den Lamellen durch Andruck in den Spannring (22) hineingeschoben wird. Im Andruckbereich sind die Lamellen (11') mit einer Kappe (23) aus einem elastischen Material abgedeckt. Auch bei dieser Ausführungsform wird somit durch die primäre Andruckbewegung des Fingers eine Sekundärbewegung quer zur primären Andruckrichtung erzeugt, die zu einem lateralen Zusammendrücken der Hautoberfläche führt. Die Erzeugung eines Körperbereiches erhöhten Innendruckes (50) ist aus Figur 4B zu erkennen. Die Kappe (23) aus elastischem Material bewirkt einerseits, daß die Haut nicht zwischen den Lamellen eingeklemmt werden kann als auch eine Anpassung der Kompressionseinheit an die Geometrie des angedrückten Körperteiles.

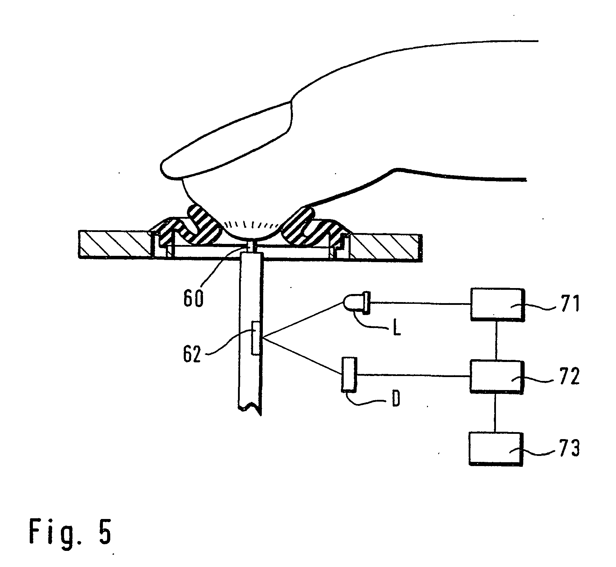

Figur 5 zeigt ein integriertes System zur Analyse von Körperflüssigkeit, das eine Kompressionseinheit gemäß Figur 1 beinhaltet. Wie bereits oben ausgeführt, weist die Kompressionseinheit eine Öffnung auf, zwischen der sich der komprimierte Bereich des Körperteiles befindet. Durch diese Öffnung in der Kompressionseinheit kann sowohl die Perforationsvorrichtung auf das Körperteil zugreifen um eine Hautöffnung zu erzeugen als auch eine Entnahme von Körperflüssigkeit erfolgen. Bei der in Figur 5 dargestellten Ausführungsform beinhaltet die Perforationsvorrichtung eine Kanüle (60), die in den komprimierten Bereich eingestochen wird und dort zum Sammeln von Körperflüssigkeit verbleibt. Die Kanüle (60) mündet in eine Analysezone (62), die im dargestellten Beispiel eine von der Analytkonzentration abhängige Farbänderung erfährt. Zur Analyse wird die Analysezone (62) mit einer Lichtquelle (L) bestrahlt und reflektierte Strahlung von einem Detektor (D) detektiert. Die Lichtquelle wird von einer Steuereinheit (71) angesteuert und das Signal des Detektors von einer Auswerteeinheit (72) ausgewertet. Vorzugsweise regelt die Auswerteeinheit (72) auch die Ansteuereinheit (71). Die Auswerteeinheit (72) führt eine Auswertung des Detektorsignales durch, um die Konzentration des in der Körperflüssigkeit enthaltenen Analyten zu ermitteln. Das Analyseergebnis wird mit einer Ausgabeeinheit (73), beispielsweise einem LC-Display, ausgegeben.

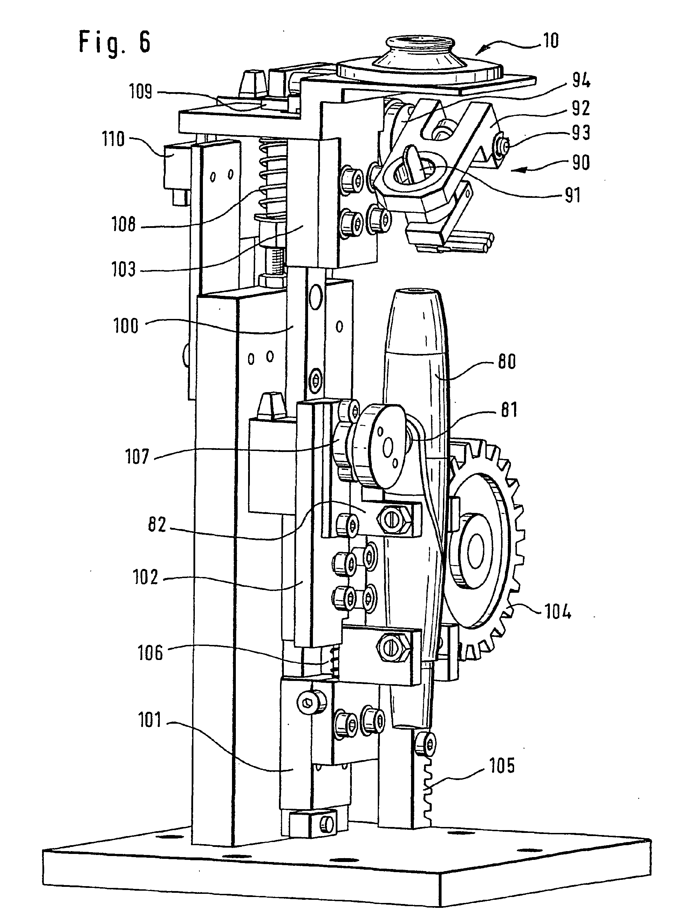

Figur 6 zeigt ein integriertes System, mit dem eine Perforation der Haut und nachfolgend eine Aufnahme von Blut zur Analyse vollautomatisch erfolgt. Das System beinhaltet drei Funktionseinheiten, nämlich eine Kompressionseinheit (10), eine Perforationsvorrichtung (80) sowie ein Analysesystem (90). Die genannten Einheiten sind verschiebbar an einer Linearführungseinheit (100) montiert. Die Linearführungseinheit besteht aus einer Führungsschiene (100) mit drei verschiebbar an ihr befestigten Schlitten (101, 102, 103). Der erste Schlitten (101) wird mittels eines Zahnrades (104) und einer Zahnstange (105) von einem Servoantrieb bewegt (in der gewählten Ansicht nicht zu sehen). Durch eine Feder (106) wird die Bewegung des ersten Schlittens (101) an den zweiten Schlitten (102) mit daran befestigter Stechhilfe (80) weitergegeben. Der Andruck der Stechhilfe an den Finger kann aufgrund der Feder mit annähernd konstanter Kraft, unabhängig vom Weg, realisiert werden. Die Kraft sollte ca. 2N nicht überschritten. bei größeren Kräften besteht die Gefahr, daß während des Andrückens und Stechens in der Fingerkuppe bereits gestautes Blut aus dem abgeschlossenen Volumen herausgedrückt wird. Die potentiell gewinnbare Blutmenge würde sich somit reduzieren. Durch einen Servoantrieb (107) mit Nockenscheibe kann ein Auslöseknopf (81) der Stechhilfe betätigt werden. Die Kompressionseinheit (10) ist auf dem dritten Schlitten (103) montiert und wird durch eine einstellbare Feder (108) von ca. 10 -15 N an einen oberen Anschlag (109) gedrückt. Durch die mit dem Finger des Benutzers aufgebrachte Kraft bewegt sich der dritte Schlitten (103) nach unten gegen einen Endschalter (110). Ohne Erreichen einer Endposition, bei dem der dritte Schlitten gegen den Endschalter gedrückt ist wird der weitere Funktionsablauf nicht freigegeben. Hierzu ist der Endschalter mit einer Ablaufsteuerung verbunden, die eine Aktivierung / Betätigung der

Systemeinheiten steuert. Das System beinhaltet weiterhin eine Analyseeinheit (90) mit einem Teststreifen (91), der in einer Halterungseinheit (92) drehbar auf einer Antriebswelle (93) gelagert ist. Die Drehmomentübertragung von Antriebswelle zu Halterungseinheit (92) erfolgt durch eine Schenkelfeder. Die Andruckkraft des Teststreifens auf den Finger ist somit annähernd unabhängig vom Drehwinkel. Die Andruckkraft beträgt ca. 1N und sollte diesen Wert nicht wesentlich übersteigen. Zu hohe Andruckkräfte verschließen zum einen den erzeugten Stichkanal und bewirken außerdem ein Verdrängen des aufgestauten Blutvolumens im abgeschlossenen, komprimierten Gewebe. Der Servoantrieb (94), der die Antriebswelle (93) antreibt ist in der gewählten Ansicht nur teilweise zu sehen.

Ablauf zum Durchführen einer Messung:

In die Perforationsvorrichtung (Stechhilfe 80) wird eine Lanzette eingesetzt, die Stechhilfe wird gespannt (kann automatisiert erfolgen). Dann wird die Stechhilfe in die Halterung (82) eingesetzt. In die Halterungseinheit (90) wird ein unbenutzter Teststreifen (91) eingesetzt.

Im Ausgangszustand befindet sich die Halterungseinheit (92) in einer Position, die eine Bewegung der Stechhilfe in Richtung der Kompressionseinheit (10) ermöglicht (weggeschwenkt). Die Stechhilfe ist zur Durchführung einer Perforation soweit in die Kompressionseinheit hineingefahren, daß durch Auflegen und Durchdrücken der Kompressionseinheit die Stirnseite der Stechhilfe vom Benutzer gefühlt werden kann. Die Andruckkraft der Stechhilfe wird in dieser Position durch die Feder (106) geregelt (ca. 2N). Die vom Benutzer aufgebrachte externe Kraft auf den Fingerkonus wird mittels Feder (108) und Endschalter (110) abgefragt und während der ganzen Messung überwacht (10 - 15 N). Ein Wegnehmen des Fingers führt zum Abbruch der Messung.

Hat der Benutzer seinen Finger optimal positioniert, kann er den Meßvorgang starten, bzw. der Meßvorgang wird automatisch gestartet.

Der Servoantrieb (107) betätigt den Auslöseknopf der Stechhilfe (80). Unmittelbar nach dem Stechvorgang wird die Stechhilfe von der Fingerkuppe weggefahren und so positioniert, daß die Analyseeinheit ungehindert bewegt werden kann. Während einer Wartezeit von 3 -10 sec tritt ein Teil des gestauten Blutvolumens aus der Stichwunde aus, und bildet einen Tropfen an der Hautoberfläche. Nach der Wartezeit wird die Analyseeinheit so an die Hautoberfläche beigeschwenkt, daß der Teststreifen mit seiner Öffnung in den Blutstropfen eintaucht. Dabei wird die maximale Andruckkraft von ca. 1 N von der Schenkelfeder geregelt. Sobald das Testfeld des Teststreifens ausreichend benetzt ist, wird dem Benutzer von der Auswerteelektronik mitgeteilt, daß er den Finger wegnehmen kann. Das gewonnene Meßergebnis wird auf einem LCD dargestellt. Alternativ zu dieser Vorgehensweise ist es auch möglich, die Analyseeinheit an die Hautoberfläche heranzufahren und dort zu verharren, um Blut aufzunehmen. In einer bevorzugten Ausgestaltung wird überwacht, ob der Teststreifen ausreichend mit Blut benetzt ist und dies kann dem Benutzer mitgeteilt werden, so daß er seinen Finger wegnehmen kann. Mit dieser Vorgehensweise kann die Zeit, die der Benutzer sein Körperteil andrücken muß, verringert werden. Bei der genannten zweiten Vorgehensweise ist es bevorzugt, den Teststreifen nicht an den Körper anzudrücken, sondern ihn lediglich nahe an die Hautoberfläche heranzuführen, so daß er von austretendem Blut benetzt wird.

## Patentansprüche

1. System zur Entnahme von Körperflüssigkeit aus einem Bereich eines Körperteils, insbesondere der Fingerbeere, mit
- einer Kompressionseinheit (10), an die das Körperteil in einer Primärrichtung angedrückt wird und die den Andruck teilweise in eine Bewegung in eine Sekundärrichtung mit Anteil quer zur Primärrichtung umwandelt, so daß eine Erhöhung des Innendruckes in einem Bereich des Körperteiles erzeugt wird,
- einer Perforationsvorrichtung (60,80), insbesondere einer Lanzette oder Kanüle, zur Erzeugung einer Körperöffnung in dem Bereich erhöhten Innendruckes, wobei die Kompressionseinheit einen Andruckbereich aus einem deformierbaren Material besitzt, **dadurch gekennzeichnet, dass** in das System ein Analysesystem (90) zur Bestimmung der Konzentration eines Analyten, insbesondere Glucose, integriert ist.

2. System gemäß Anspruch 1, bei dem der Andruckbereich eine Öffnung mit einem Rand aufweist, auf den das Körperteil gedrückt wird, wodurch sich die lichte Weite der Öffnung verkleinert.

3. System gemäß Anspruch 2, bei dem der Rand in der Form eines Ringes ausgebildet ist.

4. System gemäß Anspruch 1 oder 2, bei dem der Andruckbereich eine Öffnung aufweist, durch die die Perforationsvorrichtung in den Bereich erhöhten Innendruckes eindringen kann.

5. System gemäß Anspruch 1, bei dem der Andruckbereich aus einem Elastomer gefertigt ist.

6. System gemäß einem der vorangehenden Ansprüche, bei dem die Öffnung vor dem Andruck eine lichte Weite von 4 mm oder mehr aufweist.

7. System gemäß Anspruch 1, bei dem die Perforationsvorrichtung relativ zu der Kompressionseinheit verschiebbar angeordnet ist.

8. System gemäß Anspruch 7, bei dem die Perforationsvorrichtung federgelagert ist.

9. System gemäß Anspruch 1, 7 oder 8, bei dem die Einstechtiefe der Perforationsvorrichtung einstellbar ist.

10. System gemäß Anspruch 1 oder 7, das eine Aufnahmevorrichtung zur Aufnahme von Körperflüssigkeit besitzt.

11. System gemäß Anspruch 10, bei dem die Aufnahmevorrichtung relativ zur Kompressionseinheit verschiebbar angeordnet ist.

12. System gemäß Anspruch 1, in das eine Steuereinheit integriert ist, die die Aktivierung von Entnahmevorrichtung und Analysesystern koordiniert steuert.

13. System gemäß Anspruch 1, bei dem das Analysesystern zur Aufnahme von Körperflüssigkeit durch eine Öffnung in der Kompressionseinheit an den Bereich erhöhten Innendruckes heranbewegt wird.

14. System gemäß Anspruch 1, wobei das Analysesystern mindestens ein Testelement beinhaltet.

15. System gemäß Anspruch 10, wobei die Aufnahmevorrichtung eine Kanüle oder ein kapillaraktives Material ist und sich an die Kanüle oder das kapillaraktive Material eine Analysezone anschließt, die bei Anwesenheit des Analyten ein konzentrationsabhängiges Signal liefert.

## Claims

1. System for withdrawing body fluid from a region of a body part in particular a finger pad comprising:
- a compression unit (10) against which the body part is pressed in a primary direction and which partially converts the applied pressure into a movement in a secondary direction with a component perpendicular to the primary direction such that the internal pressure is increased in a region of the body part,
- a perforation device (60, 80), in particular a lancet or cannula, to produce a body opening in the area of the increased internal pressure, wherein the compression unit has a pressure-application region made of a deformable material, **characterized in that** an analytical system (90) for determining the concentration of an analyte, in particular glucose, is integrated into the system.

2. System according to claim 1, in which the pressure-application region has an opening with a rim on which the body part is pressed which results in a reduction in the inner width of the opening.

3. System according to claim 2, in which the rim is in the form of a ring.

4. System according to claim 1 or 2, in which the pressure-application region has an opening through which the perforation device can penetrate into the region of increased internal pressure.

5. System according to claim 1, in which the pressure-application region is made of an elastomer.

6. System according to one of the previous claims, in which the opening has an inner width of 4 mm or more before pressure is applied.

7. System according to claim 1, in which the perforation device is movably disposed relative to the compression unit.

8. System according to claim 7, in which the perforation device is spring loaded.

9. System according to claims 1, 7 or 8, in which the puncture depth of the perforation device is adjustable.

10. System according to claims 1 or 7, which has a receiving device to receive body fluid.

11. System according to claim 10, in which the receiving device is movably disposed relative to the compression unit.

12. System according to claim 1, in which a control unit is integrated which coordinatively controls the activation of the withdrawal device and analytical system.

13. System according to claim 1, in which the analytical system is moved towards the region of increased internal pressure through an opening in the compression unit in order to take up body fluid.

14. System according to claim 1, in which the analytical system contains at least one test element.

15. System according to claim 10, in which the receiving device is a cannula or a capillary-active material and an analytical zone adjoins the cannula or the capillary-active material said analytical zone yielding a concentration-dependent signal when the analyte is present.

## Revendications

1. Système de prélèvement de liquide corporel sur une zone d'une partie du corps, en particulier la pulpe des doigts, comprenant :
- une unité de compression (10) sur laquelle on presse la partie du corps dans une direction première et qui transforme la pression, partiellement, en un mouvement dans une direction secondaire avec une part perpendiculaire à la direction première, ce qui provoque un accroissement de la pression interne dans une zone de la partie du corps,
- un dispositif de perforation (60, 80), en particulier une lancette ou une canule, destiné à créer un orifice corporel dans la zone de pression interne accrue, l'unité de compression possédant une zone de pression constituée d'une matière déformable, **caractérisé en ce qu'**un système d'analyse (90) destiné à déterminer la concentration d'un analyte, en particulier de glucose, est intégré dans le système.

2. Système selon la revendication 1, dans lequel la zone de pression comporte une ouverture munie d'un rebord sur lequel on presse la partie du corps, ce qui réduit le diamètre interne de l'ouverture.

3. Système selon la revendication 2, dans lequel le rebord est réalisé sous la forme d'un anneau.

4. Système selon la revendication 1 ou 2, dans lequel la zone de pression comporte une ouverture à travers laquelle le dispositif de perforation peut pénétrer dans la zone de pression interne accrue.

5. Système selon la revendication 1, dans lequel la zone de pression est réalisée en un élastomère.

6. Système selon l'une des revendications précédentes, dans lequel l'ouverture, avant la pression, présente un diamètre intérieur de 4 mm ou plus.

7. Système selon la revendication 1, dans lequel le dispositif de perforation est disposé de manière à pouvoir être déplacé par rapport à l'unité de compression.

8. Système selon la revendication 7, dans lequel le dispositif de perforation est monté sur ressort.

9. Système selon la revendication 1, 7 ou 8, dans lequel la profondeur d'incision du dispositif de perforation est réglable.

10. Système selon la revendication 1 ou 7, lequel possède un dispositif de collecte destiné à recueillir du liquide corporel.

11. Système selon la revendication 10, dans lequel le dispositif de collecte est disposé de manière à pouvoir être déplacé par rapport à l'unité de compression.

12. Système selon la revendication 1, dans lequel est intégrée une unité de commande qui commande l'activation du dispositif de prélèvement et du système d'analyse.

13. Système selon la revendication 1, dans lequel le système d'analyse, en vue de recueillir du liquide corporel, est rapproché de la zone de pression interne accrue à travers une ouverture ménagée dans l'unité de compression.

14. Système selon la revendication 1, dans lequel le système d'analyse comporte au moins un élément de test.

15. Système selon la revendication 10, dans lequel le dispositif de collecte est une canule ou un matériel tensioactif, la canule ou le matériel tensioactif étant suivi(e) d'une zone d'analyse qui, en présence de l'analyte, délivre un signal dépendant de la concentration.
